(19) **Europäisches Patentamt** / **European Patent Office** / **Office européen des brevets**

(11) **EP 3 240 530 B1**

(12) **EUROPEAN PATENT SPECIFICATION**

(45) Date of publication and mention
of the grant of the patent:
**29.01.2020 Bulletin 2020/05**

(21) Application number: **15837177.3**

(22) Date of filing: **28.12.2015**

(51) Int Cl.:
*B02C 17/02* (2006.01)  *B02C 19/10* (2006.01)
*A61M 11/00* (2006.01)  *A61K 9/00* (2006.01)
*A61K 9/14* (2006.01)  *A61M 15/00* (2006.01)
*B07B 7/02* (2006.01)  *B02C 23/30* (2006.01)
*A61M 16/10* (2006.01)  *A61K 33/14* (2006.01)
*A61M 11/02* (2006.01)  *B02C 23/24* (2006.01)

(86) International application number:
**PCT/HU2015/000082**

(87) International publication number:
**WO 2016/108055 (07.07.2016 Gazette 2016/27)**

(54) **UNIT FOR THE MICRONIZATION AND DOSAGE OF SOLID ACTIVE AGENTS**

EINHEIT ZUR MIKRONISIERUNG UND DOSIERUNG VON FESTEN WIRKSTOFFEN

UNITÉ POUR LA MICRONISATION ET LE DOSAGE D'AGENTS ACTIFS SOLIDES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **30.12.2014 HU P1400625**

(43) Date of publication of application:
**08.11.2017 Bulletin 2017/45**

(60) Divisional application:
**19000551.2**

(73) Proprietor: **Kókai, Tamás
1126 Budapest (HU)**

(72) Inventor: **Kókai, Tamás
1126 Budapest (HU)**

(74) Representative: **Mészarosné Donusz, Katalin
S.B.G. & K. Patent and Law Offices
Andrassy ut 113.
1062 Budapest (HU)**

(56) References cited:
**EP-A2- 1 941 926        WO-A1-02/089875
WO-A1-2007/132441     DE-A1- 4 003 989
DE-A1-102012 111 431   US-A1- 2010 319 694**

## Description

Technical field

The invention relates to a unit for the micronization of a solid active agent, such as a salt, for inhalation, comprising a micronizer driven by a motor.

Devices and apparatuses adapted for direct inhalation are arrangements that are capable of introducing the active agent directly into the respiratory tract through, by way of example, a mouthpiece or a mask.

Devices and apparatuses for indirect inhalation and air treatment devices and apparatuses introduce the active agent into the air space of a room which is inhaled by the room's occupants.

Micronization is a process during which inhalable-sized particles separate from the surface of the solid / crystalline active agent, by way of example, salt, due to rubbing action and collision.

Description of prior art

The prior art that is closest to the object of the present invention is primarily related to salt therapy applications.

The incidence of respiratory tract disorders has increased significantly over the past decades. Effective treatments for such disorders include salt therapy (halotherapy). Salt utilized for halotherapy is a substance having natural antihistamine effects. If applied regularly, it prevents allergy-inducing histamines from getting activated by allergens. Since salt is one of the most effective natural anti-inflammatory substances, halotherapy may be successfully applied for cleaning both the upper and lower respiratory tracts from inflammations, viruses and pathogens. The prior art includes several salt inhaler devices. In sattrooms the salt particles released into the air of the room are ionized (have negative charge), while allergens (dust, pollens) have positive charge. Negative NaCl (salt) ions reduce the allergenicity of these positively charged particles in the cells of the respiratory system.

Patent specification RU 2,062,120 C1 discloses a salt inhaler device to which the user applies suction through a mouthpiece. The housing of the device, adapted to receive the salt, is rotatable. The inside space of the housing is divided into portions by two perforated members, with the salt crystals falling through the openings of the members when suction is applied to the mouthpiece. The housing comprises feed, suction, and inspection openings. The mouthpiece is attached to a so-called diffuser that is connected to the housing through air pipes. Connection through a diffuser provides that the fixedly arranged mouthpiece is safely attached to the rotatable housing.

Patent specification EP 1,941,926 A2 discloses an apparatus for producing salt aerosol that does not include a rotary drum. It comprises a fan, a delivery pipe connected to the output of the fan, a salt pulverizer arranged in a fixedly arranged salt chamber, and a throat adapted to produce aerosol. In the pipe section being between the output of fan and the pipe connection to the throat the delivery pipe is provided with a heating device, with a grid-type collisional ionization arrangement being disposed at the output of the delivery pipe. The aerosol is preheated to 100-150 C° by the heating device. The grinding mill pulverizes most of the salt into 0.5-5 $\mu$m -sized particles. The fan rotates at 2800 rev/min, the speed of the aerosol being approximately 27 m/s.

Patent specification DE 4,003,989 A1 discloses an apparatus for producing aerosol for treating asthma. From a structural point of view the apparatus is essentially identical to the one disclosed in the document EP 1,941,926. Salt is pulverized by a pulverizer in a fixedly arranged salt chamber, and the aerosol carrying pulverized salt is delivered into the outside air by a fan through a grid-type collider device, further reducing particle size to 0.2-20 $\mu$m. Air is preheated to approximately 600 C° by the fan applying heating elements.

The specification SU 1,741,809 A1 discloses an apparatus for producing aerosol of common salt. The apparatus comprises a fan blowing hot air through a heater unit into a fixedly arranged salt chamber where salt crystals are pulverized by a grinding mill. Salt aerosol is discharged from the apparatus through an output pipe. The flow of the aerosol may be adjusted by a baffle plate arranged at the salt chamber.

An apparatus for producing salt aerosol is disclosed in the specification RU 2,025,139 C1, where suction is applied to a salt chamber arranged at the bottom end of the outlet pipe by blowing out air by a fan. Pulverized salt is sucked out from bores disposed in the side walls of the chambers and is delivered into the outside air.

The document DE 19708862 A1 discloses a filter for cleaning exhaust gases, wherein air to be cleaned is blown through a salt layer received in a fixedly arranged container.

The document with publication number WO 2005/055984 A1 also discloses an apparatus for producing salt aerosol. The operating principle of the apparatus is based on high-energy colliding, and the apparatus does not include a rotary drum. Salt is fed through a throat and chute into a fixedly arranged grinding chamber, where crystals are ground by a grinder element. Salt aerosol is produced by compressed air fed into the grinding chamber, whence the aerosol is discharged also by means of compressed air.

The document with publication number US 2004/050860 discloses an apparatus for loosening, equally distributing, and dosing/dispensing factory-prepared pulverized active agents. The apparatus does not include a rotary drum or a filter, and does not carry out micronization.

Known dry micronization apparatuses have the common disadvantage of either being overly complex or oversized. In case of some of the known solutions the particles become agglomerated easily in moist air, significantly deteriorating operating efficiency, while means for appropriate dosage are also missing.

In other types of dosing/dispensing apparatus the problem is solved by releasing active agent doses already micronized and coated with carrier material, doses mixed and encapsulated in the factory, or by making an aerosol from the aqueous solution of the active agent.

Such a solution is explained in document US 3,971,377 which discloses an inhaler device having the primary capability of introducing solid active agents that have been pulverized and mixed with carrier material in advance in a factory, and have been put into a closed container - a capsule - into the respiratory tract such that holes having a diameter of 0.6-0.65 mm are created by piercing members on the surface of the capsule placed in the apparatus, and then the powder contained in the rotary drum receiving the capsule is introduced in the air stream of the device by spinning up the rotary drum (as if from a centrifuge with a ripped-up wall). The function of the holes, 0.6-0.65 mm in diameter, is not filtering but releasing the active agent from the capsule.

The rotary drum is not closed, it only performs the function of retaining the axis of rotation of the capsule, and is not capable of micronizing the active agent held therein.

This known solution is aimed at dispensing an active agent that already is of a particle size suitable for inhalation.

According to the solution disclosed in the patent specification US 3,971,377 a perforated atomizing disc, which is not a part of the rotary drum, can be placed in the air stream. In case of a pulverized active agent its only role is to de-agglomerate the active agent prone to clumping/agglomeration. The disc does not micronize or filter the solid active agent.

The solution according to the document US 3,971,377 is also capable of dispensing liquid active agents applying the above mentioned disc, functioning as a liquid atomizer.

According to the solution disclosed in the patent specification US 3,971,377 the active agent released from the capsule only passes through the chamber, and thereby no active agent accumulation occurs in the chamber.

The document GB 2,275,877 A discloses a liquid atomizer comprising a rotary drum, wherein liquid introduced into the drum by capillary action is forced through a tiny orifice on the wall of the high-speed rotary drum, while atomizing is carried out by the passing high-speed air stream.

The drum is not capable of micronizing or receiving a solid active agent.

The major drawback of the known solutions based on the micronization of liquids is that the size of the released droplets - originally a few microns - by the time they reach the lungs increases due to air moisture to such an extent that the droplets cannot enter the alveoli, and the method utilizing capsules can only be applied for releasing a limited number of factory-prepared doses of micronized active agent mixed with carrier material, and thereby it is unfavourable for uninterrupted dispensing and for application in devices other that direct inhalers, while the carrier material may put an unnecessary burden on the lungs and may alter the effects of the active agent.

Document WO02/089875 is also well-known in the art.

Description of the invention

The object of the present invention is to eliminate the disadvantages of known solutions and to provide a unit that, by means of rubbing and colliding against one another and against the wall of the device solid active agent grains that have a grain size similar to table salt, produces micronized active agent of the desired particle size, such as micronized salt which micronized active agent can be introduced into the surrounding air or directly into the respiratory tract by applying an inhaler apparatus or an air treatment device/apparatus comprising the unit according to the invention. Using the unit, a continuous supply of air rich in carrier-free micronized active agent with a particle size preferably lower than 5-10 microns, by way of example, micronized salt, is made available to the users, which particle size is required according to the literature for active agent particles to enter the alveoli. In case the primary objective is to make particles adhere in the upper airways for therapeutic effect, rather than introducing them into the lungs, the particle size of the active agent may reach 20 microns, or a different particle size suited for the particular therapeutic objective.

The invention is based on the recognition that by rotating or spinning up in an appropriate manner a closed rotary drum with walls at least in part made of a filtering material with an appropriate filtering capacity, such as a rigid mesh-like or microperforated filtering material, the drum receiving active agent grains having a grain size similar to table salt, a micronized active agent is produced by means of turning over and/or colliding the active agent grains, and/or rubbing them against the wall of the rotary drum, the micronized active agent being released filtered through the openings of the filtering material.

The objective of the invention is fulfilled by a unit adapted for micronizing a solid active agent, such as salt, that has a motor-driven micronizer and that is characterised in that the micronizer is a closed rotary drum receiving a solid active agent with a grain size similar to table salt, with the walls of the rotary drum being at least in part made of a material having a filtering capacity of 0.1-1000 $\mu$m, by way of example, of a rigid mesh-like or microperforated material.

In a preferred embodiment of the unit according to the invention the walls of the rotary drum are at least in part made of a material having a filtering capacity of 0.1-100 $\mu$m or 0.1-20 $\mu$m, by way of example, of a rigid mesh-like or microperforated material.

A preferred embodiment of the unit according to the invention comprises a preferably horizontal-axis rotary drum driven by a constant rotational speed motor, while another preferred embodiment comprises a rotary drum having its axis set at an arbitrary angle, the drum being spun up by the motor in a cyclical manner.

With a constant rotational speed arrangement the active agent grains, by way of example, salt grains, are being turned over inside the horizontal-axis rotary drum, thereby rubbing against one another and producing a micronized active agent that is released filtered through the openings of the filtering material.

In case of the arrangement based on the rapid change of the speed of rotation a rotary drum with an axis set at an arbitrary angle is rapidly spun up about its axis, then it is braked partially or to a halt, and is spun up again in the same or the opposite direction. During the rapid spin-up and the optional rapid deceleration phases the active agent grains, by way of example, salt grains, rub against the walls of the rotary drum, and, being turned over, against one another due to the friction caused by the wall of the rotary drum, producing a micronized active agent that is released filtered through the openings of the filtering material. In addition to that, the air flow generated by rotation removes the separated particles.

In a further preferred embodiment of the unit according to the invention a reduction gear is disposed between the motor and the rotary drum.

A further preferred embodiment of the unit according to the invention includes a fan.

In an expedient embodiment of the unit according to the invention a heating element is located below the rotary drum or in the air flow path upstream of the rotary drum, the heating element being adapted for keeping the rotary drum at a higher temperature than surrounding air. Thereby the active agent grains can be kept from getting saturated with water absorbed from air moisture, and the active agent grains - kept under drier conditions - are less prone to agglomeration. Therefore, the efficiency of micronization is improved, and the lower-moisture micronized active agent thus produced can be released easier through the openings of the filtering surface of the rotary drum.

Another expedient embodiment of the invention is built into an accumulation and dosing chamber fitted with flap valves or mechanical or electronic blocking valves, by means of which the individual dispensing of the accumulated larger doses can be provided.

A further expedient embodiment of the unit according to the invention further comprises control electronics, the control unit of the control electronics being fitted with an electronic device adapted for blocking the operation of the unit based on parameter values specified in advance and on the acquired operating data. On the one hand, the control electronics can be applied for providing accurate dosage of the micronized active agent, while on the other hand the further operation of the unit can also be blocked by it when it is necessary, for instance at the end of a pre-programmed therapy, or upon the expiry of the contained active agent.

The objective of the invention is further fulfilled by providing a unit wherein the micronizer consists of a rotary block made of the active agent, a friction block made of the material of the rotary block or of a different material and being adapted to be in frictional relation with the rotary block, and a clamping mechanism adapted for holding the friction block in position and for clamping the friction block to the rotary block, the micronizer being disposed in a closed housing, the wall surfaces of the housing being at least in part made of a material having a filtering capacity of 0.1-1000 $\mu$m, and the rotary block is preferably disc- or ring-shaped and is made preferably of salt (NaCl).

In a preferred embodiment of the unit according to the invention the wall surfaces of the housing are at least in part made of a rigid mesh-like or microperforated material.

A preferred embodiment of the unit according to the invention is incorporated in an indirect inhalation or air treatment apparatus, or in a direct inhaler apparatus, expediently in the air flow path.

The term "air treatment device/apparatus" may refer to any device or apparatus from a tabletop fan to an industrial blower.

## Brief description of the drawings

Preferred embodiments of the unit according to the present invention will be described in detail below, referring to the attached drawings, where

Fig. 1 is the schematic drawing of the first preferred embodiment of the unit according to the invention,
Fig. 2 is the schematic drawing of the second preferred embodiment of the unit according to the invention,
Fig. 3 is an exemplary arrangement for building the unit according to the invention into an indirect inhaler device,
Fig. 4 is an exemplary arrangement for building the unit according to Fig. 2 into a direct inhaler device,
Fig. 5 shows the block diagram of an exemplary control unit of the unit according to the invention, and
Fig. 6 is the schematic drawing of a further preferred embodiment of the unit according to the invention that does not include a rotary drum.

## Modes for carrying out the invention

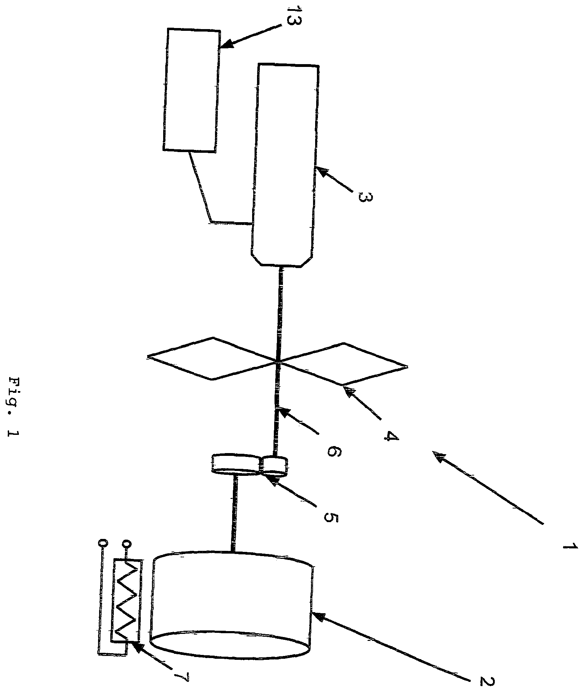

The unit according to the invention, illustrated in Fig. 1, consists of two major parts, a rotary drum 2 and a motor 3. The cylindrical surface of the rotary drum 2 is at least in a part of its surface made of a material having a filtering capacity of 0.1-100μm, preferably a closely woven metal mesh or microperforated sheet. The drum is expediently formed as a cylinder with a surface having a filtering capacity of 0.1-1000μm, but it may also have a different, e.g. a spherical shape. The filter material has a typical filtering capacity of 0.1-20 microns, but the particle size required for therapy can be different - it may be as large as 100 microns for application primarily in the upper respiratory tract.

Since there is no officially established upper limit for the particle size of inhalable dust particles floating for a limited time (according to the WHO the particle size ranges from under 1 micron to above 100 microns, with no specific upper limit), the upper limit of the particle size range let through by the filter has been specified as 1000 microns, which allows an appropriate leeway. On the other hand, with regard to the fact that the presently applied method of micronization generates extremely fine particles, having even sub-micron particle size, then - while although the most expedient maximum particle size would be e.g. 0.1-20 microns but different maximum particle size values are not harmful either - driven, for example, by economical considerations, the use of materials having coarser filtering capacity may also be allowed, which results in an active agent with a particle size primarily appropriate for the application, and also in a micronized active agent having coarser particles that settle in the environment and in the upper respiratory tract. The filtering capacity range of 0.1-1000μm has been specified based on this.

The active agent grains having the same grain size as table salt (NaCl), typically 0.1-4 mm, are placed in the rotary drum 2 in such a manner that the material does not take up the entire volume of the rotary drum 2. During the operation of the unit, fine particles of the active agent separate from the grains loaded in the rotary drum 2. The unit 1 optionally includes a fan 4. In this exemplary embodiment the fan 4 and the rotary drum 2 have a common shaft 6 driven by a motor 3. Also, in this embodiment a reducing gear 5 is disposed between the motor 3 and the rotary drum 2. The reducing gear can also be omitted. It should be noted that the rotary drum 2 and the fan 4 may be driven independently of each other.

A heating element 7, adapted for keeping the active agent loaded in the rotary drum 2 sufficiently dry during operation, is disposed below the rotary drum 2.

It has to be noted that the heating element 7 may also be omitted.

Depending on the intended use, the unit 1 can be fitted with control electronics 13, the functionality whereof will be described in detail later.

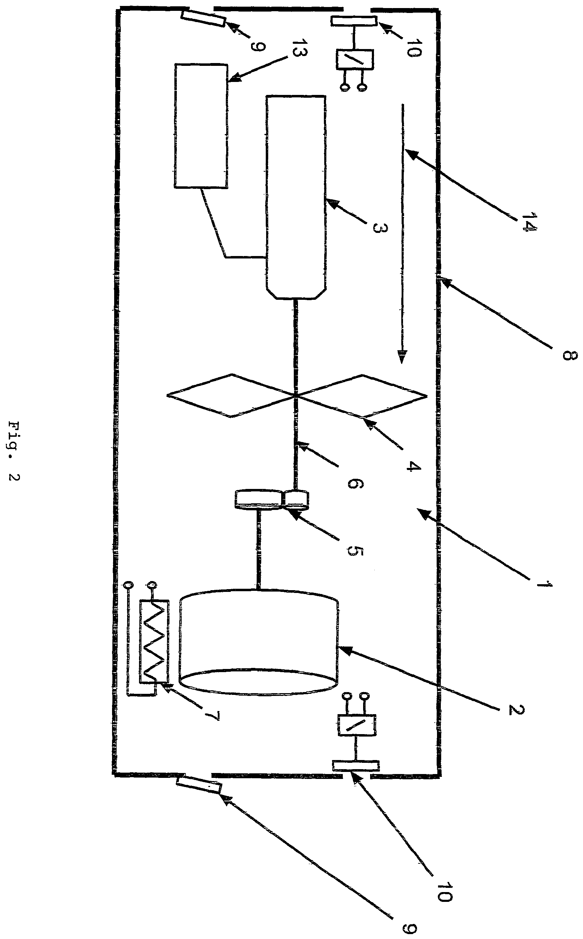

Fig. 2 illustrates an embodiment of the unit 1 shown in Fig. 1 that also comprises an accumulation and dosing chamber 8 fitted with flap valves 9 and/or electromechanical or mechanical blocking valves 10 at its input and output sides. For the sake of simplicity, both solutions have been included herein. The accumulation and dosing chamber 8 according to Fig. 2 may at the same time be the housing of an inhaler device. The unit 1 may also be fitted with control electronics 13. The arrow 14 indicates the direction of the air flow through the accumulation and dosing chamber 8.

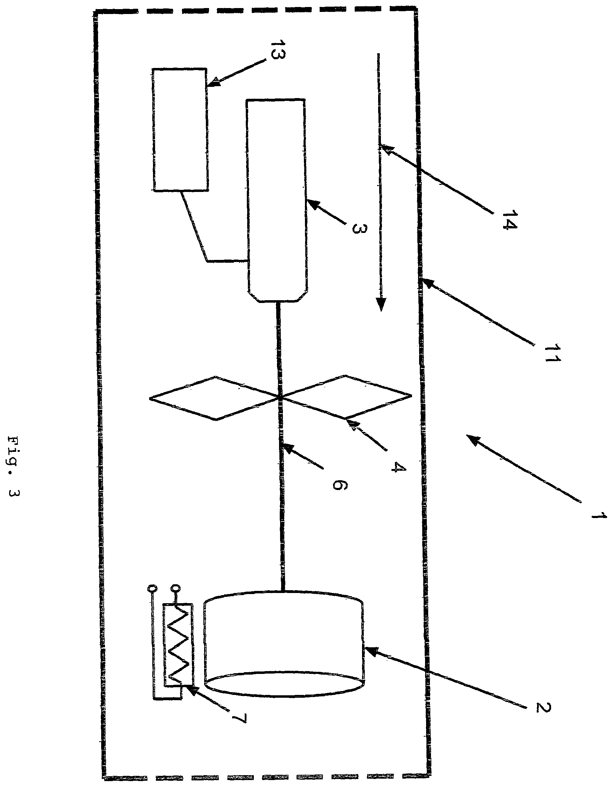

Fig. 3 shows another exemplary embodiment of the unit 1 illustrated in Fig. 1, a simple apparatus comprising a fan. The unit 1 according to the invention and the control electronics 13 thereof are disposed inside the housing 11 of the apparatus. Again, the arrow 14 indicates the direction of the air flow.

As it is seen in the drawing, in this embodiment there is no reducing gear between the rotary drum 2 and the fan 4, and thus the fan 4 and the rotary drum 2 are operating at the same rotational speed. The embodiment described above can be applied as a simple, tabletop indirect inhaler apparatus comprising a fan.

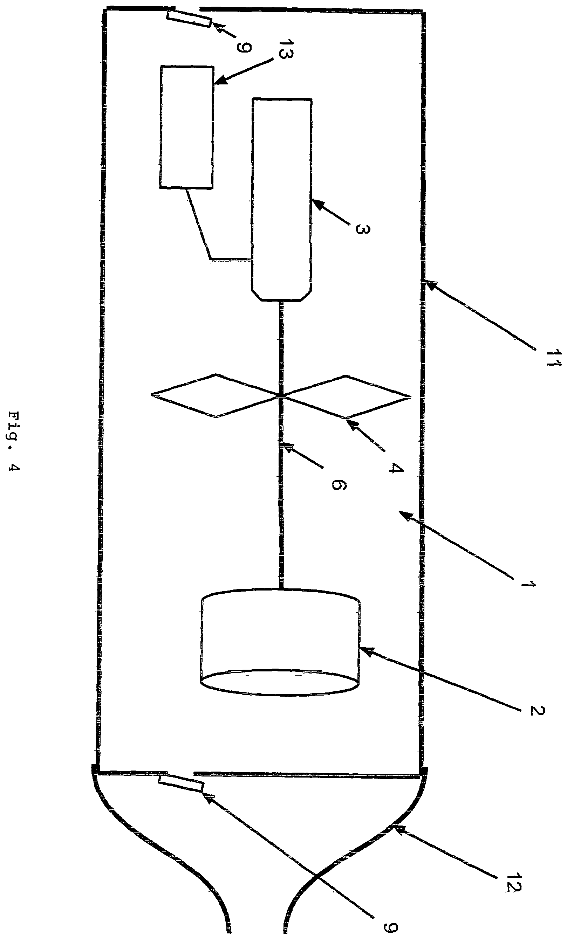

Fig. 4 illustrates another exemplary embodiment of the unit shown in Fig. 2.

In the unit 1 no reducing gear is disposed between the rotary drum 2 and the fan 4, and thereby the rotary drum 2 and the fan 4 are driven with the same rotational speed by the motor 3. The unit 1 is also equipped with control electronics 13. The unit 1 is implemented as a handheld device adapted for inhalation. A respective flap valve 9 is disposed at both ends of the housing 11 that receives the unit 1. By means of the flap valves 9 the prepared dose can be prevented from escaping from the housing before suction is applied to the adapter 12 connected to one end of the housing, and moisture can also be prevented from reentering the device through the adapter (which the users doing the inhalation place in their mouth).

It should be noted that the unit illustrated in Figs. 1 and 2 can be built into any inhaler or air treatment device/apparatus, but it is imperative that the unit 1 is located in, or is connected to, the air flow path of the inhaler device/apparatus.

The rotary drum 2 of the unit 1 illustrated in Figs. 1-4 operates either in a constant rotational speed mode or in a mode based on rapid rotational speed changes.

It has to be noted that the constant rotational speed mode is typically applied with the solution comprising a horizontal-axis gear, while the mode based on rapid rotational speed changes is typically utilized with the configuration wherein the longitudinal axis of the device is set at an arbitrary angle.

During the operation of the constant rotational speed, preferably horizontal-axis rotary drum 2 of the unit 1 the active agent grains, by way of example, salt crystals, preferably NaCl crystals, that are contained in the rotary drum 2 - but only partially fill the rotary drum 2 - are rotated about a horizontal axis 6 or an axis set at an angle of at most 75° relative to the horizontal at such a speed that the crystals are pulled downwards by the gravitational force just before they could reach the top dead point, which means that the gravitational force exerted on the crystals should exceed the centrifugal force (as calculated for a horizontal axis):

The gravitational force is:

$$F = m*g$$

The centrifugal force is:

$$F = m*(r*4*pi^2)/T^2$$

Hence

$$g = r*4*pi^2/T^2_{min}$$

$$1/T^2_{min} = g/(r*4*pi^2)$$

$$T_{min} = \sqrt{r*4*pi2/g}$$ . Considering that

pi2/g≈1, the approximate formula is $T_{min} ≈ 2*\sqrt{r}$.

This implies that the rotation cycle time should be longer than that, where g refers to gravitational acceleration, and r denotes a value equalling the inside radius of the rotary drum minus the radius of the smallest grain. In practice, it is expedient to allow an appropriate leeway.

The present embodiment has a rotary drum 2 with a diameter of 3.1 cm, the rotary drum 2 being rotated at a maximum speed of n = 244 rev/min during the phase when micronizing is carried out applying the above principle. As indicated by practical experience, the angle of the axis 6 of rotation may be varied in a relatively wide range - $\alpha = \pm 15°$ -, without significantly degrading the efficiency of the apparatus.

The rotary drum 2 is rotated at a constant speed, and thereby the active agent grains contained therein are turned over, colliding and rubbing against one another, and thus a micronized active agent is produced that leaves the rotary drum through its filtering material having a filtering capacity of 0.5-1000 $\mu$m. In addition to gravity, expelling the micronized active agent is also assisted by the centrifugal forces occurring upon rotation of the drum, the optionally included fan 4 and/or by the suction effect of the air flow generated by the inhaler or air treatment device/apparatus in which the unit 1 is disposed. A sufficiently low rotational speed of the rotary drum 2 can also be provided by the reducing gear 5.

The unit 1 according to the invention can also be operated without a reducing gear (see Fig. 3).

The unit 1 may also be operated (with an arbitrary angle of axis) by spinning up the rotary drum 2 by means of the motor 2 in the same direction or in alternating directions. By "spinning up" it is meant that the rotary drum 2 is rotated with varying speed, i.e. the rotary drum 2 is rotated such that it has a high angular acceleration, which is followed by braking it either actively or taking advantage of mechanical losses, thereby providing that, in addition to the micronizing action described above, the active agent grains are also subjected to a rubbing and turning action generated by the high initial acceleration and fast braking of the rotary drum 2. In this case, in a resting state the active agent grains inside the rotary drum 2 take a position determined by gravity. During the spin-up phase - due to their inertia - the grains only gradually reach the rotational speed of the rotary drum 2, meanwhile being rubbed against the wall of the rotary drum 2 and turned over and rubbed against one another, and spreading all over the cylindrical surface of the rotary drum 2, thereby producing a micronized active agent that leaves the drum in a filtered manner through the 0.1-1000 $\mu$m filtering-capacity walls of the rotary drum 2. A similar phenomenon occurs during hard braking. The expelling of the micronized active agent is assisted by the centrifugal forces occurring upon rotation of the drum, the optionally included fan 4 and/or by the suction effect of the air flow generated by the inhaler or air treatment device/apparatus in which the unit 1 is disposed, and, depending on the angle of the principal axis, also by gravity. Applying such a mode of operation with the dimensions exemplified above, the spin-up duration can be 1.5 sec., the braking duration 3.5 s, and the length of the rest period can be 2 s. If a half filled-up rotary drum 2 having a 3.1 cm-long horizontal axis is spun up from resting state, the angular acceleration of the rotary drum 2 is preferably 25 $1/s^2$. Rest periods are expediently included because of the variability of the duration of the deceleration/braking phase.

It is important to note that by the term "motor" an electric motor is primarily meant but it can also refer to a functionally equivalent rotating mechanism with a different operating principle. The above described modes of operation can be partly realized by mechanisms operated exclusively by the input of mechanical energy (by way of example, by pressing a button connected to a ratchet and gear mechanism, or by winding up a coil spring). These mechanisms can mostly be utilized when built into portable inhalers, by way of example in such a manner that the user spins up the rotary drum as many times and with a frequency as specified in the user's manual. The implementation of the mechanical rotating mechanism is not included in the invention since it can be realized by a skilled person in a number of known ways. The functionality of the motor may also be performed by the motor or fan of the apparatus containing the unit according to the invention.

It is of common knowledge that when a hygroscopic material is heated to a higher-than ambient temperature, its moisture content will drop. Taking into account that a major part of solid active agents to be micronized, for example NaCl, is hygroscopic, and therefore prone to clumping, it is expedient to heat it in order to keep it drier. This can be achieved by heating the rotary drum 2 and thereby the active agent contained therein applying a heating element 7. The heating element 7 is disposed below the rotary drum 2 (considering the typical operating position of the rotary drum 2). For a rotary drum 2 with a diameter of 3.1 cm and a length of 3 cm, containing 6 grams of NaCl crystals the required heating power of the heating element is 1-2 Watts, in case the air flow is applied intermittently (corresponding to the spin-up phases). It should be noted here that the heating element may also be disposed upstream of the rotary drum.

In Fig. 2 a variant of the unit 1 according to the invention further comprising an accumulation and dosing chamber 8 is shown, wherein the accumulation and dosing chamber 8 essentially corresponds to the housing that contains the unit's mechanism. Due to the action of the flap valves and blocking valves the particles leaving the rotary drum will be kept inside the accumulation and dosing chamber 8 while a dose is being prepared, and until the produced micronized active agent is used up. A dose of the active agent can leave the accumulation and dosing chamber 8 on the one hand when the user inhales the prepared micronized active agent by means of an oral or nasal adapter 12 (see Fig. 4) such that due to the suction action the flap valves 9 free up the path of the air flow (and thereby, the path of the active agent). At the same time, the flap valves prevent moist and contaminated exhaled air - resulting from user error - from reentering the accumulation and dosing chamber 8 of the unit 1. Alternatively (by way of example, with a larger-sized inhaler unit) the active agent can also be fed to the air flow of the inhaler device containing the unit expediently by opening the electromechanical or mechanical blocking valves 10 and making the air flow through them. The optionally included fan 4 facilitates the expelling of the micronized active agent from the rotary drum 2, and also allows for circulating the micronized active agent in a floating state in the accumulation and dosing chamber 8 until it is used, preventing the micronized agent from settling.

The application of the accumulation and dosing chamber is justified if either the user cannot be expected to inhale low doses for a prolonged period of time (for example, with a pocketable handheld electronic inhaler), or if a single, higher dose is preferred for other reasons. Then, the duration of dose preparation (performed expediently in a closed chamber) is relatively long.

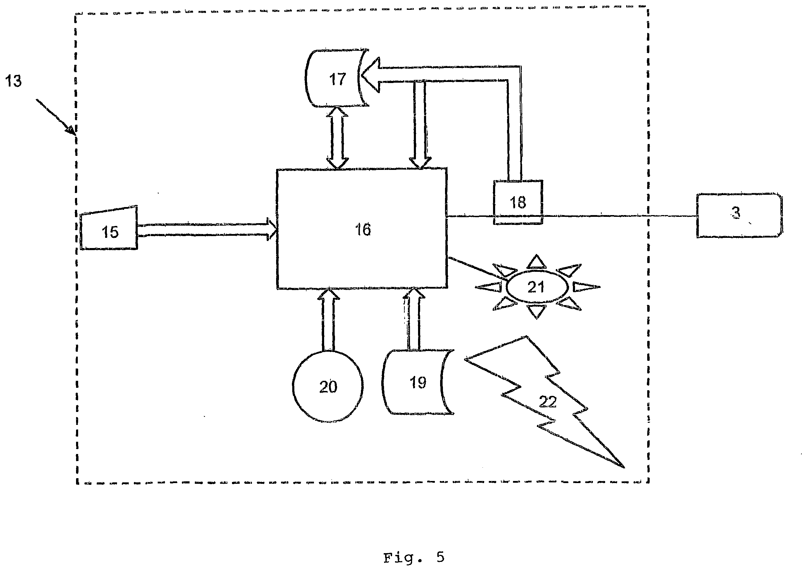

Fig. 5 shows the flow diagram of the control electronics 13 applicable with the unit 1 according to the invention. The control electronics can be operated by a switch 15, and has a control unit 16 and a power electronics unit.

A storage unit 17, adapted to store all the operating data that have been gathered since the unit 1 was first switched on, is connected to the control unit 16. Such data are the operating parameters of the motor 3, the count of operating cycles, and the data supplied by an ammeter 18 that may also be fed directly to the control unit 16. Control of the apparatus is performed by the control unit 16 utilizing current ammeter 18 data and data acquired earlier from the ammeter 18, further data stored in the storage unit 17, and also the data retrieved from a storage unit 19.

The storage unit 19 is applied for storing lab-measured decrease characteristics of the applied agent, the date of expiry, the maximum cycle count, and other factory-set parameters required for process control and for blocking the operation of the apparatus.

The control unit 16 also comprises a built-in clock 20. The control unit also comprises a signalling unit 21 adapted to provide light and/or sound signalling to indicate the on-off switching of the apparatus and for instance the expiry of the contained agent.

The control electronics 13 may also comprise a separate power source 22 (battery). By controlling the micronizer utilizing an embodiment or a combination of more than one embodiments of the unit 1 according to the invention in an appropriate manner it can be provided with a sufficient accuracy that the desired amount of micronized active agent is produced during each operating cycle.

In order to do that, the momentarily available (remaining) quantity of the active agent has to be determined applying one of the following methods:

- Under laboratory conditions the active agent release (per time unit) characteristics of the unit, dependent on certain parameters, such as the operating voltage, the mode of operation, the amount of remaining active agent, are determined and permanently stored in the memory of the unit at the factory. Based on these characteristics, and also on the stored operating data of former operating cycles and the starting amount the remaining amount of active agent can be calculated.

- Considering that in case of the mode of operation based on rapid change of rotational speed the spin-up characteristics will change as a function of the remaining amount of active agent (spinning up a lower mass requires a lower initial current), the remaining amount of active agent can also be inferred from the initial current.

Knowing the remaining amount of active agent the cycle time and optionally other parameters, by way of example the operating voltage of the motor, are automaticatty adjusted by the control unit 16.

The solutions operating as described above can be combined with a solution wherein - under certain conditions - the control unit 16 blocks the further operation of the unit.

This solution is based on that the conditions under which the amount of delivered agent falls below the desired minimum within a fixed operation period are known from lab measurements, or under which a dose cannot be prepared within a comfortable period or with a sufficient safety margin even with the application of the operating parameters adjusted by the control electronics. In that case, and also upon the expiry of the active agent or at the end of a pre-programmed therapy session, the operation of the unit is blocked by the control electronics.

In the mode of operation of the unit according to the invention that is based on rapid changes of rotational speed the duration of the operating cycles (detectable by listening) - the cycles consisting of a spin-up phase, a total or partial braking phase, and optionally, a resting phase - is expediently adjusted to the cycle time of relaxed breathing, i.e., approximately 6 seconds, which according to the literature can be utilised for practicing relaxed breathing in order to achieve proper air exchange and a lower blood pressure.

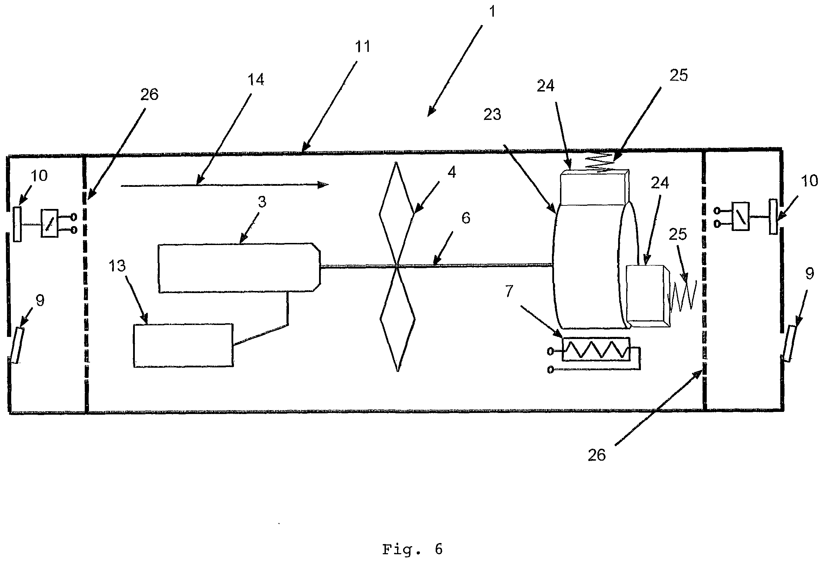

Fig. 6 schematically illustrates a further conceivable embodiment of the unit according to the invention that does not include a rotary drum. The unit 1 consists of four major parts: a motor 3, a rotary block 23 - preferably a disc or a ring - a friction block 24 adapted to be in frictional relation with the rotary block 23, and a clamping device 25 adapted for pressing the friction block 24 against the rotary block 23. The unit 1 is disposed in an accumulation and dosing chamber 8 that can be fitted with flap valves 9 and blocking valves 10.

The unit is preferably incorporated in a housing 11, the input and output portions of the housing 11 being covered by a material having a filtering capacity of 0.1-1000μm, preferably a closely woven filter 26, preferably a metallic mesh or microperforated plate adapted for filtering the air flowing through the unit 1.

The rotary block 23 - a disc or ring - is disposed on the shaft 6 of the fan 4 driven by the motor 3, and thereby the rotary block 23 is driven by the motor 3 together with the fan.

A heating element 7 may be disposed below the rotary block 23. The motor 3 may also have control electronics 13.

The rotary block 23 and the friction block 24 can be made of identical or different materials.

If the rotary block 23 and the friction block 24 are made of the same material, then the material is, by way of example, table salt (NaCl). In case they are made of different materials, the rotary block 23 can be made, by way of example, of NaCl, while the material of the friction block 24 can be, by way of example, salbutamol.

The functionality of the heating element 7, the control electronics 13, the accumulation and dosing chamber 8, the flap valve 9 and the blocking valve 10 is essentially the same as was explained in relation to the unit comprising a rotary drum.

The operation of the unit 1 shown in Fig. 6 is described as follows:
By switching on the motor 3 the fan 4 and the rotary block 23 - disc or ring - are brought into operation. During the rotation of the disc or ring 23 the friction block 24, being in frictional relation with the disc or ring 23, separates particles therefrom, the particles being expelled through the perforations of the filter 26 constituting the closing wallings of the housing 11 into the surrounding environment by the air flow produced by the fan 4, where its beneficial effect is produced upon inhaling them by the user.

Due to the frictional relation of the rotary block 23 - disc or ring - and the friction block 24 fine particles separate from both objects.

The micronization capacity of the unit 1 is fundamentally dependent on rotational speed.

It should be noted that particle separation may also be accomplished applying a mechanism different from the friction block 24.

The unit according to the invention has the following advantages:

- it has simple structural arrangement,
- it can be adapted to suit different user needs.

**Claims**

1. Unit (1) for the micronization of a solid active agent to be micronized for inhalation purposes, such as a salt, preferably table salt (NaCl), comprising a micronizer driven by a motor (3), wherein micronization is performed by a unit constituted by a closed rotary drum (2) containing a solid active agent having the same grain size, preferably 0.1-4 mm, as table salt (NaCl), where the wall surfaces of the micronizer unit are made at least in part_of a material having a filtering capacity of 0.1-1000 μm, the material preferably being a mesh-like or microperforated material.

2. The unit according to Claim 1, **characterised in that** at least a portion of the wall surfaces of the rotary drum (2) is made of a material having a filtering capacity of 0.1-100 μm, the material preferably being a rigid mesh-like or microperforated material.

3. The unit according to Claim 1, **characterised in that** at least a portion of the wall surfaces of the rotary drum (2) is made of a material having a filtering capacity of 0.1-20 μm, the material preferably being a rigid mesh-like or microperforated material.

4. The unit (1) according to any one of Claims 1-3, **characterised in that** the rotary drum (2) is driven by a constant rotational speed motor (3), the longitudinal axis of the rotary drum (2) being preferably horizontal or set at an angle not exceeding 75° with respect to the horizontal.

5. The unit (1) according to any one of Claims 1-3, **characterised in that** the rotary drum (2) is spun up by the motor (3) in a cyclical manner, the rotary drum (2) preferably having a horizontal longitudinal axis, having a diameter of 31 mm, and being half filled-up, the resting rotary drum (2) preferably undergoing an accelerating rotary motion with an angular acceleration of 25 1/s$^2$.

**6.** The unit according to any one of Claims 1-5, **characterised in that** a heating element (7) is included under the rotary drum (2) or in the air path upstream of the rotary drum (2).

**Patentansprüche**

**1.** Einheit (1) zur Mikronisierung eines zu mikronisierenden festen Wirkstoffs zu Inhalationszwecken, zum Beispiet eines Salzes, bevorzugt von Speisesalz (NaCl), umfassend einen von einem Motor (3) angetriebenen Mikronisierer, wobei die Mikronisierung von einer Einheit durchgeführt wird, die aus einer geschlossenen Drehtrommel (2) besteht, die einen festen Wirkstoff mit der gleichen Korngröße, bevorzugt 0,1-4 mm, wie Speisesalz (NaCl) enthält, wobei die Wandoberflächen der Mikronisierereinheit mindestens teilweise aus einem Material mit einer Filterkapazität von 0,1-1000 μm hergestellt sind, wobei das Material bevorzugt ein netzartiges oder mikroperforiertes Material ist.

**2.** Einheit nach Anspruch 1, **dadurch gekennzeichnet, dass** mindestens ein Abschnitt der Wandoberflächen der Drehtrommel (2) aus einem Material mit einer Filterkapazität von 0,1-100 μm hergestellt ist, wobei das Material vorzugsweise ein starres netzartiges oder mikroperforiertes Material ist.

**3.** Einheit nach Anspruch 1, **dadurch gekennzeichnet, dass** mindestens ein Abschnitt der Wandoberflächen der Drehtrommel (2) aus einem Material mit einer Filterkapazität von 0,1-20 μm hergestellt ist, wobei das Material vorzugsweise ein starres netzartiges oder mikroperforiertes Material ist.

**4.** Einheit (1) nach einem der Ansprüche 1-3, **dadurch gekennzeichnet, dass** die Drehtrommel (2) von einem Motor (3) mit konstanter Drehzahl angetrieben wird, wobei die Längsachse der Drehtrommel (2) bevorzugt horizontal ist oder in einem Winkel von nicht mehr als 75° in Bezug auf die Horizontale eingestellt ist.

**5.** Einheit (1) nach einem der Ansprüche 1-3, **dadurch gekennzeichnet, dass** die Drehtrommel (2) von dem Motor (3) in einer zyklischen Weise drehbeschleunigt wird, wobei die Drehtrommel (2) bevorzugt eine horizontale Längsachse aufweist, einen Durchmesser von 31 mm aufweist und halb gefüllt ist, wobei die ruhende Drehtrommel (2) bevorzugt eine beschleunigende Drehbewegung mit einer Winkelbeschleunigung von 25 1/s$^2$ erfährt.

**6.** Einheit (1) nach einem der Ansprüche 1-5, **dadurch gekennzeichnet, dass** ein Heizelement (7) unter der Drehtrommel (2) oder in dem Luftweg stromaufwärts der Drehtrommel (2) beigefügt ist.

**Revendications**

**1.** Unité (1) pour la micronisation d'un agent actif solide à microniser à des fins d'inhalation, tel qu'un sel, de préférence un sel de table (NaCl), comprenant un équipement de micronisation entraîné par un moteur (3), dans laquelle la micronisation est effectuée par une unité constituée d'un tambour rotatif (2) fermé contenant un agent actif solide présentant la même taille de grain, de préférence de 0,1-4 mm, que le sel de table (NaCl), où les surfaces de paroi de l'unité de micronisation sont constituées au moins en partie d'un matériau présentant une capacité de filtrage de 0,1-1000 μm, le matériau étant de préférence un matériau de type maille ou un matériau microperforé.

**2.** Unité selon la revendication 1, **caractérisée en ce que** au moins une partie des surfaces de paroi du tambour rotatif (2) est constituée d'un matériau présentant une capacité de filtrage de 0,1 à 100 μm, le matériau étant de préférence un matériau de type maille rigide ou un matériau microperforé.

**3.** Unité selon la revendication 1, **caractérisée en ce qu'**au moins une partie des surfaces de paroi du tambour rotatif (2) est constituée d'un matériau présentant une capacité de filtrage de 0,1 à 20 μm, le matériau étant de préférence un matériau de type maille rigide ou un matériau microperforé.

**4.** Unité (1) selon l'une quelconque des revendications 1-3, **caractérisée en ce que** le tambour rotatif (2) est entraîné par un moteur (3) à vitesse de rotation constante, l'axe longitudinal du tambour rotatif (2) étant de préférence horizontal ou réglé à un angle ne dépassant pas 75° par rapport à l'horizontale.

**5.** Unité (1) selon l'une quelconque des revendications 1-3, **caractérisée en ce que** le tambour rotatif (2) est tourné vers le haut par le moteur (3) d'une manière cyclique, le tambour rotatif (2) présentant de préférence un axe longitudinal horizontal, présentant un diamètre de 31 mm, et étant à moitié rempli, le tambour rotatif (2) au repos subissant de préférence un mouvement rotatif d'accélération avec une accélération angulaire de 25 1/s$^2$.

**6.** Unité selon l'une quelconque des revendications 1-5, **caractérisée en ce qu'**un élément chauffant (7) est inclus sous le tambour rotatif (2) ou dans le trajet d'air en amont du tambour rotatif (2).

Fig. 1

Fig. 2

Fig. 3

Fig. 4

14

Fig. 5

Fig. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- RU 2062120 C1
- EP 1941926 A2
- DE 4003989 A1
- EP 1941926 A
- SU 1741809 A1
- RU 2025139 C1
- DE 19708862 A1
- WO 2005055984 A1
- US 2004050860 A
- US 3971377 A
- GB 2275877 A
- WO 02089875 A